# EUROPÄISCHE PATENTANMELDUNG

(11) **EP 1 134 574 A2**
(43) Veröffentlichungstag der Anmeldung: **19.09.2001**
(21) Anmeldenummer: 01103907.0
(22) Anmeldetag: 17.02.2001
(51) Int. Cl.: G01N 9/04

(54) **Vorrichtung zur Überprüfung der in einen Tank eines Kraftfahrzeuges eingefüllten Kraftstoff-Sorte**

(30) Priorität: 14.03.2000 DE 10012252
(71) Anmelder: Bayerische Motoren Werke Aktiengesellschaft, 80809 München (DE)
(72) Erfinder: Lorenz, Martin, 85399 Hallbergmoos (DE)

(57) **Zusammenfassung**

Die Erfindung betrifft eine Vorrichtung zur Überprüfung der in einen Tank eines Kraftfahrzeuges eingefüllten Kraftstoff-Art, wobei das Gewicht eines definierten Volumens des zugeführten Kraftstoffes bestimmbar ist. Vorgesehen ist hierfür eine vom Kraftstoff-Einfüllrohr abzweigende und in einem mit einem Überlauf versehenen Überprüf-Behälter mündende Befüll-Leitung. Bevorzugt wird das Gewicht des definierten Volumens oder des befüllten Überprüf-Behälters elektronisch mittels eines Drucksensors bestimmt, auf welchem sich dieses definierte Volumen oder der Überprüf-Behälter abstützt. Bei einer signifikanten Abweichung des ermittelten Gewichts vom Sollgewicht kann eine Inbetriebnahme des mit dem Kraftstoff gespeisten Fahrzeug-Antriebsaggregates verhindert werden. Allgemein ist die Vorrichtung auch an anderen Maschinen zur Überprüfung der in deren Tank eingefüllten Betriebsflüssigkeit anwendbar.

## Beschreibung

Die Erfindung betrifft eine Vorrichtung zur Überprüfung der in einen Maschinen-Tank eingefüllten Betriebsflüssigkeit, insbesondere der in einen Tank eines Kraftfahrzeuges eingefüllten Kraftstoff-Art, worunter insbesondere Diesel-Kraftstoff einerseits und Ottomotor-Kraftstoff, d.h. Benzin andererseits zu verstehen ist. Zum technischen Umfeld wird lediglich beispielshalber auf die DE 27 49 335 A1 verwiesen.

Bekanntermaßen kann eine Falschbetankung erhebliche Schäden an einem Kraftfahrzeug-Antriebsaggregat, bspw. Ottomotor, hervorrufen, wenn nämlich versehentlich Diesel-Kraftstoff in den Fahrzeug-Tank eingefüllt wird und der Motor danach in Betrieb genommen wird. Auch der umgekehrte Fall, nämlich der Betrieb eines Dieselmotors mit Benzin (Ottomotor-Kraftstoff) ist nur bis zu einem bestimmten Benzinanteil in einem dem Motor zugeführten Diesel-Benzin-Kraftstoffgemisch möglich, einen höheren Benzinanteil in einem solchen Gemisch gilt es unbedingt zu vermeiden.

Im Übrigen kann sich die gleiche Problematik allgemein bei der Befüllung eines Maschinen-Tankes mit einer Betriebsflüssigkeit oder dgl. stellen, d.h. es kann erwünscht sein, die Tank-Befüllung für eine Maschine einfach und schnell hinsichtlich Richtigkeit des gewählten und eingefüllten Betriebsstoffes oder dgl. überprüfen zu können.

Mit der vorliegenden Erfindung soll eine Vorrichtung aufgezeigt werden, mit Hilfe derer - insbesondere in einem Kraftfahrzeug installiert - die in den Tank eingefüllte Kraftstoff-Art (bzw. allgemein: Maschinen-Betriebsflüssigkeit) einfach auf Richtigkeit überprüft werden kann (= Aufgabe der Erfindung). Die Lösung dieser Aufgabe ist dadurch gekennzeichnet, daß das Gewicht eines definierten Volumens des zugeführten Kraftstoffes bestimmbar ist. Vorteilhafte Aus- und Weiterbildungen sind Inhalt der Unteransprüche

Erfindungsgemäß wird bevorzugt ein Teil der bei einem Betankvorgang zugeführten Kraftstoffmenge/Betriebsflüssigkeit gewogen, und zwar ein bestimmtes, definiertes Volumen derselben. Die Erfindung macht sich dabei die Tatsache zunutze, daß sich die verschiedenen Kraftstoff-Arten / Betriebsflüssigkeiten auch hinsichtlich ihres spezifischen Gewichtes bzw. ihrer Dichte unterscheiden. Beispielsweise hat Benzin ein spezifisches Gewicht von ca. 0,75 kg/dm³, während dasjenige von Diesel-Kraftstoff in der Größenordnung von 0,83 bis 0,84 kg/dm³ liegt. Wird nun während des Betankens des Kraftfahrzeuges eine Teilmenge von eingefülltem Kraftstoff bevorzugt in einen separaten Überprüf-Behälter abgezweigt und wird dieser ein definiertes Volumen von bspw. 0,5 dm³ aufweisende Überprüf-Behälter im vollständig befüllten Zustand gewogen, so kann anhand des ermittelten Gewichtes mit Sicherheit festgestellt werden, ob bspw. Diesel-Kraftstoff oder Ottomotor-Benzin eingefüllt wurde. Dabei kann diese Überprüfung bereits während des Betankungsvorganges durchgeführt werden, so daß dieser im Falle einer Falschbetankung nach Abgabe eines entsprechenden Signals baldmöglichst abgebrochen werden kann.

Ausdrücklich sei darauf hingewiesen, daß die Bestimmung des Gewichtes eines definierten zugeführten Kraftstoff-Volumens keineswegs nur durch Wägung und Ermittlung eines Wertes in Gramm erfolgen muß, sondern daß - wie bei elektrisch/elektronischer Wägung üblich - auch eine geeignete Druckmessung, Kraftmessung oder Wegmessung durchgeführt werden kann. Selbstverständlich kann eine erfindungsgemäße Vorrichtung unter Zuhilfenahme aller möglicher heute üblicher elektrischer und/oder elektronischer Hilfsmittel arbeiten, d.h. bevorzugt kann das Gewicht des definierten Volumens oder des bereits genannten befüllten Überprüf-Behälters elektronisch mittels eines Drucksensors bestimmt werden, auf welchem sich dieses definierte Volumen oder der Überprüf-Behälter abstützt.

In diesem Zusammenhang können auch Zusatzfunktionen an einer erfindungsgemäßen Vorrichtung vorgesehen sein. So kann bei Erkennung einer Falsch-Betankung, d.h. bei Erkennen einer signifikanten Abweichung des ermittelten Gewichts vom Sollgewicht nicht nur ein Alarmsignal abgegeben werden, sondern es kann dann auch eine Inbetriebnahme des mit dem Kraftstoff gespeisten Fahrzeug-Antriebsaggregates elektronisch gesteuert verhindert werden. Gleiches gilt selbstverständlich auch allgemein, d.h. bei falsch betankter Betriebsflüssigkeit kann eine Inbetriebnahme der Maschine unterbunden werden. Im Anwendungsfall bei einem Kraftfahrzeug ist es dabei möglich, dem Betreiber des Kraftfahrzeuges zu gestatten, diese Verhinderungs-Sperre zu überwinden, d.h. der Fahrer kann bspw. zunächst aufgefordert werden, nochmals zu überprüfen, ob er die richtige Kraftstoffsorte getankt hat. Bestätigt er dies ausdrücklich, so kann die Inbetriebnahme des Antriebsaggregates (Motors) ermöglicht werden. Erkennt der Fahrer jedoch, daß er tatsächlich die falsche Kraftstoff-Art eingefüllt hat, so kann der Fahrzeug-Tank leergepumpt werden, so daß sicher ein Motor-Schaden vermieden wird.

Wie eine erfindungsgemäße Vorrichtung grundsätzlich aufgebaut sein kann, wird im folgenden anhand eines bevorzugten Ausführungsbeispieles in vereinfachter Darstellung gemäß der einzigen **Figur** beschrieben. Erfindungswesentlich können dabei sämtliche näher beschriebenen Merkmale sein.

Mit der Bezugsziffer 1 ist ein gemäß der dargestellten Pfeilrichtung 10 zu einem nicht gezeigten Fahrzeug-Tank führendes Kraftstoff-Einfüllrohr bezeichnet. Von diesem Einfüllrohr 1, über welches gemäß der dargestellten Pfeilrichtung 10 bei einem Betanken des Kraftfahrzeuges Kraftstoff in den Tank eingefüllt wird, zweigt eine sog. Befüll-Leitung 2 ab, die zu einem sog. Überprüf-Behälter 3 führt. Eine geeignete Nase 4 im Bereich der Abzweigung der Befüll-Leitung 2 vom Einfüllrohr 1 stellt sicher, daß ein Teil des eingefüllten Kraftstoffs in diese Befüll-Leitung 2 gelangt.

Vorgesehen ist eine vom Überprüf-Behälter 3 von dessen geodätisch tiefstem Punkt abzweigende Entleerungsleitung 5, die letztlich im Kraftstofftank und hier im Einfüllrohr 1 mündet. In dieser Entleerungsleitung 5 ist ein Absperrventil 9 vorgesehen, welches - ausgehend von einem leeren Überprüf-Behälter 3 - zunächst geschlossen sei. Wird nun - ebenfalls ausgehend von einem leeren Überprüf-Behälter 3 - das Kraftfahrzeug betankt, so gelangt ein Teil des Kraftstoffs über die Befüll-Leitung 2 in den Überprüf-Behälter 3, und zwar solange, bis dieser vollständig befüllt ist. Weiterer danach durch die Befüll-Leitung 2 zugeführter Kraftstoff wird über einen Überlauf 6, der vom geodätisch höchsten Punkt des Überprüf-Behälters 3 bzw. von der Mündungsstelle der Befüll-Leitung 2 in diesen abzweigt, zurück in das Einfüllrohr 1 geführt.

Der ein definiertes Volumen von bspw. 0,5 Liter (dm³) aufweisende Überprüf-Behälter 3 stützt sich mit seinem Gewicht auf einem Drucksensor 7 ab, der somit als elektronische Waage fungiert. Es ist somit möglich, das Gewicht des befüllten Überprüf-Behälters 3 zu ermitteln, und da dessen Leergewicht selbstverständlich bekannt ist, auch das Gewicht des in den Überprüf-Behälter 3 eingefüllten Kraftstoffvolumens. Der zugehörige Rechenvorgang wird dabei in einer elektronischen Steuereinheit 8 durchgeführt.

In dieser elektronischen Steuereinheit 8 ist auch abgelegt, welches Gewicht ein mit dem richtigen, vom Kraftfahrzeug-Antriebsaggregat benötigten Kraftstoff befüllter Überprüf-Behälter 3 haben muß. Weicht das ermittelte bzw. gemessene Gewicht signifikant von diesem vorgegebenen Wert ab, so ist dies ein Hinweis darauf, daß möglicherweise ein falscher Kraftstoff (d.h. bspw. Diesel-Kraftstoff anstelle Benzin) betankt wird oder wurde. Daraufhin kann die Steuereinheit 8 entsprechend reagieren, d.h. ein Warnsignal abgeben und/oder eine Inbetriebnahme des Antriebsaggregats unterbinden.

Erkennt die erfindungsgemäße Vorrichtung, daß mit höchster Wahrscheinlichkeit die richtige Kraftstoff-Art oder -Sorte getankt wurde, so sind keine besonderen Maßnahmen zu ergreifen. Vielmehr kann dann mit einem folgenden Start des Kraftfahrzeuges das Absperrventil 9 geöffnet werden, so daß die gewichtsmäßig bestimmte Kraftstoffmenge aus dem Überprüf-Behälter 3 über die Entleerungsleitung 5 in den Tank gelangen kann. Anschließend wird das Absperrventil 9 wieder geschlossen, so daß die erfindungsgemäße Vorrichtung für den nächsten Tankvorgang bereit ist.

Selbstverständlich können eine Vielzahl von Details insbesondere konstruktiver Art durchaus abweichend vom gezeigten Ausführungsbeispiel gestaltet sein, ohne den Inhalt der Patentansprüche zu verlassen. Stets ergibt sich eine einfache Vorrichtung, mit Hilfe derer die Richtigkeit der getankten Kraftstoff-Sorte oder -Art auf einfache Weise festgestellt bzw. überprüft werden kann, wobei auch andere Flüssigkeiten, bspw. Wasser oder dgl. mit vom Sollwert abweichendem spezifischen Gewicht diagnostiziert werden können, und eine erfindungsgemäße Vorrichtung auch an beliebigen Tanksystemen anderer Maschinen eingesetzt werden kann.

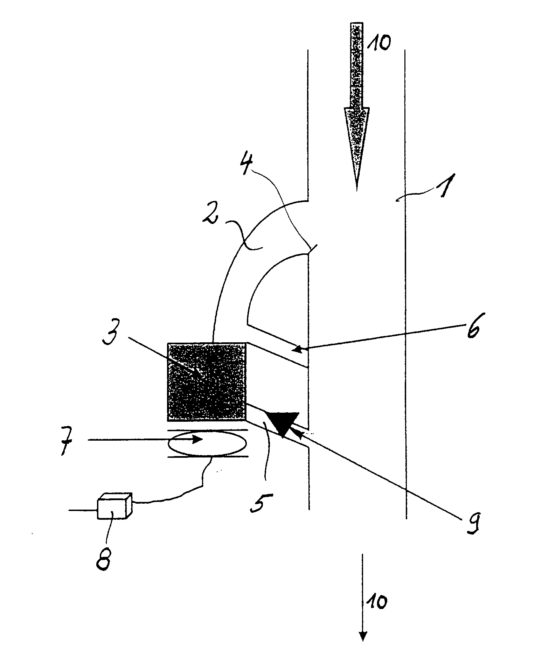

## Patentansprüche

1. Vorrichtung zur Überprüfung der in einen Maschinen-Tank eingefüllten Betriebsflüssigkeit, insbesondere der in einen Tank eines Kraftfahrzeuges eingefüllten Kraftstoff-Art, wobei das Gewicht eines definierten Volumens der zugeführten Betriebsflüssigkeit, insbesondere Kraftstoffes bestimmbar ist.

2. Vorrichtung nach Anspruch 1,
**gekennzeichnet durch** eine vom Kraftstoff-Einfüllrohr (1) abzweigende und in einem mit einem Überlauf (6) versehenen Überprüf-Behälter (3) mündende Befüll-Leitung (2).

3. Vorrichtung nach Anspruch 1 oder 2,
**dadurch gekennzeichnet, daß** das Gewicht des definierten Volumens oder des befüllten Überprüf-Behälters (3) elektronisch mittels eines Drucksensors (7) bestimmbar ist, auf welchem sich dieses definierte Volumen oder der Überprüf-Behälter (3) abstützt.

4. Vorrichtung nach einem der vorangegangenen Ansprüche,
**dadurch gekennzeichnet, daß** bei einer signifikanten Abweichung des ermittelten Gewichts vom Sollgewicht eine Inbetriebnahme der Maschine, insbesondere des mit dem Kraftstoff gespeisten Fahrzeug-Antriebsaggregates, verhindert wird.

5. Vorrichtung nach einem der vorangegangenen Ansprüche,
**gekennzeichnet durch** eine letztlich im Kraftstofftank mündende, absperrbare Entleerungsleitung (5) für den Überprüf-Behälter (3).
